# EUROPEAN PATENT SPECIFICATION

(11) **EP 1 010 429 B1**
(45) Date of publication and mention of the grant of the patent: **29.10.2003**
(21) Application number: 99124745.3
(22) Date of filing: 13.12.1999
(51) Int. Cl.: A61K 35/78

(54) **Pharmaceutical composition, in particular for dietetical use comprising a derivative of pyruvic acid and unripe bitter orange extract**
Arzneimittel, insbesondere zur diätetischen Verwendung, das Brenztraubensäurederivate und ein Extrakt der unreifen Pomeranze enthält
Composition pharmaceutique, en particulier pour l'utilisation diététique contenant un dérivé d'acide pyruvique et un extrait d'oranges amères vertes

(30) Priority: 11.12.1998 IT TO981037
(43) Date of publication of application: 21.06.2000
(73) Proprietor: ROEDER 1956 FARMACEUTICI S.p.A., 10126 Torino (IT)
(72) Inventor: Villa, Claudio, 10131 Torino (IT)
(74) Representative: Gerbino, Angelo

(56) References cited:
- WO-A-98/14200
- DATABASE BIOSIS [Online] BIOSCIENCES INFORMATION SERVICE, PHILADELPHIA, PA, US; 1994 IVY JOHN L ET AL: "Effects of pyruvate on the metabolism and insulin resistance of obese Zucker rats." Database accession no. PREV199497177700 XP002178291 & AMERICAN JOURNAL OF CLINICAL NUTRITION, vol. 59, no. 2, 1994, pages 331-337, ISSN: 0002-9165

## Description

The present invention relates to a pharmaceutical composition, in particular for dietary use, comprising at least one derivative of pyruvic acid, in particular a pyruvate or a product of the reaction of pyruvic acid with an amino-acid, and unripe bitter orange (Citrus aurantium) extract.

This composition has metabolic and thermogenic action, since it is active in relation to the regulation of the metabolic cell functions and is usable in particular in the treatment of excess body weight.

Pyruvates represent the connecting link between the most important metabolic cycles responsible for the production of cell energy. They result from the metabolism of carbohydrates, in fact constituting the final product of glycolysis, that is, the metabolic process which is responsible for the use of glucose for the production of energy, and which takes place in the cell cytoplasm. The pyruvates are then transformed, by oxidative decarboxylation, from the pyruvate-dehydrogenase enzymatic complex, into acetyl coenzyme A which falls within the Krebs cycle.

The Krebs cycle, which takes place within particular cell compartments, that is, the mitochondria, performs a key role in metabolism. It gives rise to most of the chemical energy in the form of ATP, which is used by the cell to perform its own work. By means of a series of complex reactions, the Krebs cycle permits biosynthesis of high-energy compounds and of reduced coenzymes which are re-oxidized in the electron-transfer chain with which the oxidative phosphorylation responsible for the synthesis of ATP is associated. The Krebs cycle in turn affects another metabolic process, beta-oxidation, which is responsible for the burning of fats for energy purposes. It is thus clear that the Krebs cycle represents the meeting point of several metabolic routes and can influence the above-mentioned processes, such as the respiratory chain, oxidative phosphorylation and beta-oxidation, all of which take place within the mitochondria (Medicamenta, seventh edition, Vol II, p. 535-549, Cooperativa Farmaceutica, 1992-93).

Increased availability of pyruvates increases the production of acetyl coenzyme A and consequently the activity of the Krebs cycle and of the respiratory chain, with greater utilization of the lipid substrates for the production of cell energy. The preferential burning of fats within the mitochondria also takes place in the presence of glucose. The function of the pyruvates thus consists in stimulating mitochondrial respiration, promoting the oxidation of fats rather than their storage (Stanko R.T. and Arch J.E. International Journal of Obesity, 1996, 20:925-930).

It is also probable that the presence of these compounds with 3 active carbon atoms activates or increases the activity of alternative cell cycles such as the pyruvatophosphoenol pyruvate cycle, the execution of which has a high energy cost (Stanko R.T. and Arch J.E. International Journal of Obesity, 1996, 20:925-930).

Unripe bitter orange extract also performs an important function in lipid metabolism. The extract obtained from the still unripe fruit is in fact particularly rich in biogenic amines: sinephrine, tyramine, n-methyl tyramine, octopamine and ordenine. The biogenic amines are substances of amino-acid derivation which can have different physiological effects in relation to the receptor structure with which they interact.

Unripe bitter orange extract normally contains no less than 4% of sinephrine and no less than 6% of the five amines mentioned above. Investigations carried out have demonstrated the ability of unripe bitter orange extract to stimulate lipolysis and thermogenesis in a natural manner at the level of the adipose tissue. The desired effect of the extract is attributed to the specific interaction of the amines it contains with the beta3 receptors located predominantly in the adipose tissue. The beta3 receptors in fact represent the cell structures which are responsible for the regulation of lipolysis and thermogenesis. (Astrup A. et al., Am. J. Clin. Nutr., 1992, 55: 246S-8S).

Unripe bitter orange extract therefore acts exclusively on localized accumulations of fat, promoting burning, with the consequent production of energy in the form of heat.

By virtue of the synergy of its two components, both of which are involved in cell energy metabolism, the composition of the present invention can encourage the burning of fats and discourage their storage, both in a generalized manner within the organism and in a localized manner at the level of the adipose tissue.

Pyruvic acid derivatives in fact stimulate the preferential use of fats rather than sugars for the production of cell energy in all of the cells of the organism, whereas unripe bitter orange extract promotes metabolism, and in particular thermogenesis, only at the level of the adipose tissue. The composition of the invention thus favours loss of body weight in all regions of the organism and, in particular, at the level of the accumulations of fat localized in the adipose tissue.

The pyruvic acid derivative of the composition of the invention is a pyruvate or a product of the reaction of pyruvic acid with an amino-acid.

For example, glycine may be used as the amino-acid, producing pyruvyl glycine as the reaction product.

With regard to the pyruvates, they are preferably selected from the group consisting of magnesium, calcium, sodium and potassium pyruvates and mixtures thereof. Magnesium pyruvate is the most preferred pyruvate.

The derivative of pyruvic acid and the unripe bitter orange extract are advantageously present in the composition of the invention in quantities corresponding to a ratio by weight within the range between 1:1 and 500:1 and more preferably between 1:1 and 20:1. A ratio of 4:1 is most preferred.

For the purposes of effectiveness in use, it is appropriate, again preferably, to use a minimum daily dose of 40 mg of unripe bitter orange extract, with reference to an average individual weighing 60 kg, and a minimum daily dose of 1 g of pyruvic acid derivatives, again with reference to an average individual weighing 60 kg.

The composition of the invention may also comprise any pharmaceutically and dietetically acceptable excipient and possibly further active ingredients.

The composition may be administered in any conventional manner, for example, orally, topically, or transdermally, and preferably in doses of the order of 450 mg/die of unripe bitter orange extract and 2 g/die of pyruvic acid derivatives, with reference to an average individual weighing 60 kg.

### TOXICOLOGICAL TESTS

The large number of clinical tests which have been carried out on pyruvates, even at high daily doses of the order of 15-20 g, have not shown any side effects. In particular, no abnormality affecting the nitrogen balance, the urine, or the biochemical profile of any single individual has been found. (Stanko R.T. et al., Am. J. Clin. Nutr. 1992, 56:630-5).

With regard to unripe bitter orange extract, an acute toxicity study performed on rats showed that the LD50 value, as a result of oral consumption, was more than 10g of raw material per kg of body weight of the animal. (Douds D.A., M.S., Springborn Laboratories, Inc. (SLI), Health and Environmental Sciences 640 North Elisabeth Street, Spencerville, Ohio 45887, 1997).

The extract, in the doses advised in accordance with the present invention and due to its content of biogenic amines, represents a safe product, without side effects or contraindications, which is incapable of stimulating other receptor structures such as alphal, alpha2, beta1, beta2, involved in regulating blood pressure and heart rate. Moreover, the amines present in unripe bitter orange extract - again with reference to the advised dosages - are incapable of crossing the blood-brain barrier and thus causing adverse repercussions on the activity of the central nervous system.

### CLINICAL STUDIES ON THE PYRUVATES/BITTER ORANGE COMBINATION

An investigation was performed with the purpose of confirming the effectiveness of the combination of pyruvic acid derivative and unripe bitter orange extract and their synergy of action. 4 groups of clearly overweight patients were considered: the first group was given a composition of the invention, the second group was given the same composition without the unripe bitter orange extract fraction, the third group was given the same composition without the pyruvate fraction, and the fourth group was given a composition in which both of the active ingredients were replaced by metabolically inert excipients.

The compositions of the various types of tablets given to the patients forming the various groups are given in Table I. The placebo contained, in addition to inert excipients, some vitamins and a mineral, in dietary doses having no effect on the properties of the composition of the invention. The dosage followed provided for 3 tablets per day to be taken by patients weighing up to 60 kg, 4 tablets per day by patients weighing up to 80 kg, and 5 tablets per day by patients weighing more than 80 kg.

**TABLE 1**

| 1st group | |
|---|---|
| unripe bitter orange extract | 150 mg |
| Mg pyruvate | 667 mg |
| vitamin B1 | 0.7 mg |
| vitamin B6 | 0.6 mg |
| natural vitamin C | 20 mg |
| chromium | 16.6 µg |

| 2nd group | |
|---|---|
| Mg pyruvate | 667 mg |
| vitamin B1 | 0.7 mg |
| vitamin B6 | 0.6 mg |
| natural vitamin C | 20 mg |
| chromium | 16.6 µg |

| 3rd group | |
|---|---|
| unripe bitter orange extract | 150 mg |
| vitamin B1 | 0.7 mg |
| vitamin B6 | 0.6 mg |
| natural vitamin C | 20 mg |
| chromium | 16.6 µg |

| 4th group (placebo) | |
|---|---|
| vitamin B1 | 0.7 mg |
| vitamin B6 | 0.6 mg |
| natural vitamin C | 20 mg |
| chromium | 16.6 µg |

The duration of the test was six weeks; during the first three weeks, the patients also followed a controlled diet, whereas in the last three weeks the diet was no longer subjected to controls. After six weeks, a reduction in body weight was found in all of the groups but the most significant reduction was found in the patients of the group which took the composition of the invention, in whom the weight loss affected only the fatty mass.

Haematological tests showed a more significant reduction in total cholesterol and in triglycerides only in the group which took the composition of the invention. Other parameters considered - such as glycaemia, azotaemia, creatinaemia, electrolytes, bilirubinaemia, SGOT and SGPT transaminase, alkaline phosphatase, albumin, total proteins, transferrin, thyroxin, free thyroxin index, triiodothyronine and captation index - on the other hand showed no significant differences between the 4 groups under investigation.

During the last three weeks, the group treated with the composition of the invention showed a lesser tendency to regain the weight previously lost.

In summary, therefore, the pyruvic acid derivative/unripe_ bitter orange extract combination was found to be effective in a reduction in body weight involving only the fatty mass.

The composition of the invention was also able to inhibit the slowing of the metabolism and the regaining of the lost kilos which normally occur after a controlled-calory diet has been followed.

Haematological examination and urine analysis showed no statistically significant differences between the four groups, confirming the absolute tolerability and safety of the composition of the invention.

By virtue of the synergic action of its constituents, the composition of the invention thus increases weight loss, reducing exclusively the fatty mass, helps to prevent the slowing of the metabolism which accompanies slimming treatment, and opposes the appearance of the "yo-yo" effect (a phenomenon which is manifested by a regaining of body weight when a restrictive diet is stopped).

This latter property was displayed in the last three weeks of the investigation during which a decidedly lesser tendency to regain body weight was found in the patients of the first group in comparison with the other groups.

Naturally, the principle of the invention remaining the same, the details of implementation and forms of embodiment may be varied widely with respect to those described purely by way of example, without thereby departing from its scope. In particular, these embodiments may comprise uses of the composition of the invention in therapeutic fields other than the dietary field.

## Claims

1. A pharmaceutical composition, in particular for dietary use, comprising at least one derivative of pyruvic acid and unripe bitter orange (citrus aurantium) extract.

2. A composition according to Claim 1, in which the pyruvic acid derivative is a pyruvate or a product of the reaction of pyruvic acid with an amino-acid.

3. A composition according to Claim 2, in which the pyruvate is selected from the group consisting of magnesium, calcium, sodium and potassium pyruvates and mixtures thereof.

4. A composition according to Claim 2, in which the pyruvic acid derivative is pyruvyl glycine.

5. A composition according to any one of the preceding claims, in which the at least one pyruvic acid derivative and the unripe bitter orange extract are present in quantities corresponding to a ratio by weight within the range between 1:1 and 500:1, preferably between 1:1 and 20:1, and even more preferably a ratio of 4:1.

6. A composition according to any one of the preceding claims, also comprising pharmaceutically and dietetically acceptable excipients and/or other active ingredients.

7. A composition according to any one of the preceding claims, in a form which can be administered orally, topically or transdermally.

## Patentansprüche

1. Pharmazeutische Zusammensetzung, insbesondere für diätetischen Gebrauch, mindestens umfassend ein Derivat der Brenztraubensäure und ein Extrakt unreifer Bitterorangen (citrus aurantium).

2. Zusammensetzung nach Anspruch 1, in der das Brenztraubensäurederivat ein Pyruvat oder ein Produkt der Reaktion von Brenztraubensäure mit einer Aminosäure ist.

3. Zusammensetzung nach Anspruch 2, in der das Pyruvat aus der Gruppe bestehend aus Magnesium-, Calcium-, Natrium- und Kaliumpyruvaten und Mischungen derselben ausgewählt ist.

4. Zusammensetzung nach Anspruch 2, in der das Brenztraubensäurederivat Pyruvylglycin ist.

5. Zusammensetzung nach einem der vorangehenden Ansprüche, in der das mindestens eine Brenztraubensäurederivat und das Extrakt der unreifen Bitterorange in Mengen vorhanden sind, die einem Gewichtsverhältnis im Bereich von 1:1 bis 500:1, vorzugsweise von 1:1 bis 20:1 und meistbevorzugt in einem Verhältnis von 4:1 entsprechen.

6. Zusammensetzung nach einem der vorangehenden Ansprüche, ebenfalls umfassend pharmazeutisch und diätetisch akzeptable Trägerstoffe und/oder andere Wirkstoffe.

7. Zusammensetzung nach einem der vorangehenden Ansprüche in einer Form, die oral, topisch oder transdermal verabreicht werden kann.

## Revendications

1. Composition pharmaceutique, en particulier pour une utilisation diététique, comprenant au moins un dérivé d'acide pyruvique et un extrait d'oranges non mûres *(citrus aurantium*).

2. Composé selon la revendication 1, dans lequel le dérivé d'acide pyruvique est un pyruvate ou un produit issu de la réaction de l'acide pyruvique avec un acide aminé.

3. Composition selon la revendication 2, dans laquelle le pyruvate est choisi dans le groupe constitué des pyruvates de magnésium, de calcium, de sodium et de potassium et des mélanges de ceux-ci.

4. Composition selon la revendication 2, dans laquelle le dérivé d'acide pyruvique est la pyruvyl-glycine.

5. Composition selon l'une quelconque des revendications précédentes, dans laquelle le au moins un dérivé d'acide pyruvique et l'extrait d'oranges amères non mûres sont présents dans des quantités correspondant à un rapport en poids situé dans la plage entre 1:1 et 500:1, de préférence entre 1:1 et 20:1 et de manière encore davantage préférée, correspondant à un rapport de 4:1.

6. Composition selon l'une quelconque des revendications précédentes, comprenant en outre des excipients acceptables du point de vue pharmaceutique et diététique et/ou d'autres ingrédients actifs.

7. Composition selon l'une quelconque des revendications précédentes, sous une forme qui peut être administrée oralement, topiquement ou transdermiquement.
